# EUROPÄISCHE PATENTSCHRIFT

(11) **EP 3 335 049 B1**
(45) Veröffentlichungstag und Bekanntmachung des Hinweises auf die Patenterteilung: **11.03.2026**
(21) Anmeldenummer: 16775453.0
(22) Anmeldetag: 12.08.2016
(51) Int. Cl.: G01N 33/74

(54) **ANALYSE VON IGF-BINDUNGSPROTEINEN ZUR BESTIMMUNG VON TIERWOHL**
ANALYSIS OF IGF-BINDING PROTEINS FOR DETERMINATION OF ANIMAL WELFARE
ANALYSE DE PROTÉINES SE LIANT À L'IGF POUR LA DÉTERMINATION DU BIEN-ÊTRE ANIMAL

(30) Priorität: 14.08.2015 DE 102015113502
(43) Veröffentlichungstag der Anmeldung: 20.06.2018
(73) Patentinhaber: Forschungsinstitut für Nutztierbiologie, 18196 Dummerstorf (DE)
(72) Erfinder: HÖFLICH, Andreas, 18276 Gülzow-Prüzen (DE); WIRTHGEN, Elisa, 18195 Tessin (DE)
(74) Vertreter: Moré, Solveig Helga
(86) Internationale Anmeldenummer: PCT/DE2016/100361
(87) Internationale Veröffentlichungsnummer: WO 2017/028842

(56) Entgegenhaltungen:
- A J ROBERTS ET AL: "Circulating insulin-like growth factor I, insulin-like growth factor binding proteins, growth hormone, and resumption of estrus in postpartum cows subjected to dietary energy restriction.", JOURNAL OF ANIMAL SCIENCE, vol. 75, no. 7, 1 January 1997 (1997-01-01), US, pages 1909, XP055321476, ISSN: 0096-0837, DOI: 10.2527/1997.7571909x
- "Protein Protocols Handbook, The", vol. 0, 19 February 2002, HUMANA PRESS, New Jersey, ISBN: 978-1-59259-169-5, article DICKINSON JOANNE ET AL: "Quantification of Proteins on Western Blots Using ECL", pages: 429 - 437, XP055815088, DOI: 10.1385/1-59259-169-8:15
- WIRTHGEN ELISA ET AL: "Analysis of bioactive IGF-binding proteins by quantitative western ligand blotting in different biological fluids for biomarker research", ENDOCRINE ABSTRACTS, 17 April 2014 (2014-04-17), XP055815143, DOI: 10.1530/endoabs.35.P678
- MCCUSKER ET AL: "Controlling Insulin-Like Growth Factor Activity and the Modulation of Insulin-Like Growth Factor Binding Protein and Receptor Binding", JOURNAL OF DAIRY SCIENCE, AMERICAN DAIRY SCIENCE ASSOCIATION, US, vol. 81, no. 6, 1 June 1998 (1998-06-01), pages 1790 - 1800, XP027045697, ISSN: 0022-0302, [retrieved on 19980601]
- T. LAEGER ET AL: "Effects of parturition and feed restriction on concentrations and distribution of the insulin-like growth factor-binding proteins in plasma and cerebrospinal fluid of dairy cows", JOURNAL OF DAIRY SCIENCE, vol. 97, no. 5, 1 May 2014 (2014-05-01), US, pages 2876 - 2885, XP055321897, ISSN: 0022-0302, DOI: 10.3168/jds.2013-7671
- B. W GALLAHER ET AL: "Ontogenic differences in the nutritional regulation of circulating IGF binding proteins in sheep plasma", EUROPEAN JOURNAL OF ENDOCRINOLOGY, vol. 126, no. 1, 1 January 1992 (1992-01-01), GB, pages 49 - 54, XP055321475, ISSN: 0804-4643, DOI: 10.1530/acta.0.1260049
- M J DAUNCEY ET AL: "Regulation of insulin-like growth factor binding proteins in young growing animals by alteration of energy status", GROWTH REGULATION, 1 September 1993 (1993-09-01), SCOTLAND, pages 198 - 207, XP055322629, Retrieved from the Internet <URL:http://www.ncbi.nlm.nih.gov/pubmed/7693100> [retrieved on 20161124]
- ROBERT H. MCCUSKER ET AL: "The Insulin-Like Growth Factor-Binding Proteins of Porcine Serum: Endocrine and Nutritional Regulation*", ENDOCRINOLOGY, vol. 125, no. 1, 1 July 1989 (1989-07-01), US, pages 501 - 509, XP055322408, ISSN: 0013-7227, DOI: 10.1210/endo-125-1-501
- WHITE M E ET AL: "Insulin-like growth-factor binding protein (IGFBP) serum levels and hepatic IGFBP-2 and -3 mRNA expression in diabetic and insulin-treated swine (Sus scrofa)", COMPARATIVE BIOCHEMISTRY AND PHYSIOLOGY. B. COMPARATIVEBIOCHEMISTRY, PERGAMON PRESS, LONDON, GB, vol. 106, no. 2, 1 October 1993 (1993-10-01), pages 341 - 347, XP023792154, ISSN: 0305-0491, [retrieved on 19931001], DOI: 10.1016/0305-0491(93)90311-R
- IBT GMBH ET AL: "Western-Ligand Blotting Kit for Human and Other Species IGFBP's: IBT Systems", 11 February 2010 (2010-02-11), XP055322919, Retrieved from the Internet <URL:http://www.ibtsystems.de/products/blotting-kits/western-ligand-blotting-kit-for-human-and-other-species-igfbps/product/western-ligand-blotting-kit-for-human-and-other-species-igfbps.html> [retrieved on 20161124]
- CHRISTINE HÖFLICH: "BIOMARKERS IN CANCER RESEARCH", 29 April 2014 (2014-04-29), XP055322927, Retrieved from the Internet <URL:http://www.ligandis.de/fileadmin/user_upload/downloads/2014_04_29_Ligandis_Expose_english.pdf> [retrieved on 20161124]
- WENDY C WARD ET AL: "Pediatric Research - Dexamethasone-Induced Abnormalities in Growth and Bone Metabolism in Piglets Are Partially Attenuated by Growth Hormone with No Synergistic Effect of Insulin-Like Growth Factor-I", PEDIATRIC RESEARCH, vol. 44, no. 2, 1 August 1998 (1998-08-01), pages 215 - 221, XP055322658
- CHRIS KNIGHT: "New biomarkers for monitoring animal welfare", SISVET, SOFIVET, PERUGIA, 17 June 2015 (2015-06-17), XP055322929, Retrieved from the Internet <URL:http://www.dairycareaction.org/uploads/2/4/2/6/24266896/sisvet_perugia_ck.pdf> [retrieved on 20161124]

## Beschreibung

Die Erfindung betrifft Verfahren und Kits zur Bestimmung von Tierwohl, also Tiergerechtheit oder "animal welfare". Insbesondere stellt die Erfindung ein Verfahren zur Analyse der Expression von IGF- (Insulin-Like Growth Factor) Bindungsproteinen, insbesondere IGFBP-2 und IGFBP-3, als Marker für Tierwohl zur Verfügung. Dabei lässt sich durch die Untersuchung dieser Marker zwischen kurz- und langfristiger Beeinträchtigung des Tierwohls unterscheiden, also zwischen Beeinträchtigungen, die kurzfristig beim Transport von Tieren entstehen, oder von Beeinträchtigungen, die z.B. durch Krankheit und/oder mangelhafte Haltungsbedingungen entstehen.

Die Mehrheit der Deutschen wünscht sich die Sicherheit, dass das Fleisch auf dem Teller von einem Tier stammt, welches zu Lebzeiten gut versorgt wurde. Ein Ausdruck dieses breiten Konsenses schlägt sich in der gegenwärtigen Initiative "Tierwohl" nieder. Die Teilnehmer dieser Initiative verpflichten sich dazu, bestimmte Standards wie Platzbedarf, Tageslicht, Stallklima- oder Tränke-Monitoring einzuhalten. Der komplexe Begriff Tierwohl (auch Wohlbefinden, engl. animal welfare, oder Tiergerechtheit) beschreibt den Zustand physischer und mentaler Gesundheit eines Individuums und dessen Fähigkeit, auf Umweltreize situativ zu reagieren und diese zu bewältigen (Broom, 1986). Indikatoren für ein schlechtes Wohlbefinden können eine verkürzte Lebensspanne, beeinträchtigtes Wachstum, verminderte Reproduktionsleistung, Verletzungen, Krankheiten oder Verhaltensanomalien sein. Einige dieser Merkmale können bereits vor Ort erkennbar sein. Dennoch kann das Tierwohl auch gemindert sein, ohne dass eine äußerlich erkennbare Beeinträchtigung des Tieres vorliegt (Broom, 1991).

Vom britischen Farm Animal Welfare Council (FAWC) wurde in den 80er Jahren das Konzept der "5 Freiheiten" entwickelt. Es bildet die Grundlage für verschiedene Mess- und Bewertungssysteme für Tierwohl. Das Konzept der 5 Freiheiten bezieht die genannten Wertesysteme ein und bietet einen Ansatz zur Operationalisierung, also zur praktischen Messung von Tierwohl in der Tierhaltung. Die 5 Freiheiten sind:
- Freiheit von Hunger und Durst: Tiere haben Zugang zu frischem Wasser und gesundem und gehaltvollem Futter.
- Freiheit von haltungsbedingten Beschwerden: Tiere haben eine geeignete Unterbringung (z. B. einen Unterstand auf der Weide), adäquate Liegeflächen etc.
- Freiheit von Schmerz, Verletzungen und Krankheiten: Die Tiere werden durch vorbeugende Maßnahmen, bzw. schnelle Diagnose und Behandlung, Verzicht auf Amputationen (bzw. Betäubung) versorgt.
- Freiheit von Angst und Stress: Durch Verfahren und Management werden Angst und Stress vermieden z. B. durch Verzicht auf Treibhilfen.
- Freiheit zum Ausleben normaler Verhaltensmuster: Die Tiere haben die Möglichkeit das Normalverhalten auszuüben z. B. durch ausreichendes Platzangebot, Gruppenhaltung etc.

Die Messung der verschiedenen Aspekte des Tierwohls erfolgt üblicherweise auf der Basis von Indikatoren. Hierbei wird unterschieden in:
- Ressourcenbezogene Indikatoren, die z.B. Informationen über Haltungsverfahren und Platzangebot bereitstellen
- Managementbezogene Indikatoren, die Praktiken wie die Enthornung von Rindern oder die Kastration von männlichen Ferkeln erfassen, aber auch die Fütterung und den Umgang mit den Tieren.
- Tierbezogene Indikatoren, die direkt am Tier gemessen werden. Beispiele für tierbezogene Indikatoren (animal based indicators) sind Fußballenentzündungen (Pododermatitis) bei Mastgeflügel, Lahmheiten bei Milchkühen sowie Lungenbefunde am Schlachtkörper bei Mastschweinen (wikipedia).

Tierwohl ist vor allem auch ein gesellschaftlich geprägter Begriff. Deutlich wird das am Beispiel eines Freilandschweins mit Sonnenbrand. Die Freilandhaltung entspringt dem menschlichen Ideal, der Sonnenbrand als tierbezogener Parameter ist jedoch die maßgebliche Reaktion oder Antwort des Tieres zur Bewertung oder Anpassung des Ideals. Hierzu muss der Sollwert tierbezogener Parameter ermittelt und deren Auslenkung bei Störungen untersucht werden.

In der Literatur wurde z.B bereits die Konzentration von IGF-1 in Milch (Ruffer, 2003) als Marker für Eutergesundheit diskutiert. Die Eignung des IGF-I Systems zur Prognose der Gesundheit und Fertilität bei pluriparen Milchkühen wurde von Mysegades (2014) untersucht. Walker et al. (2008) diskutieren eine Beurteilung des Intensität des Estrus bei Milchkühen als Indikator für chronischen Stress.

Biomarker als tierbezogene Indikatoren könnten einen Beitrag dazu leisten, Tierwohl zukünftig besser zu beurteilen. Dabei stellt sich auch oft die Frage, welche Faktoren zu einer Beeinträchtigung des Tierwohls führen. In Anbetracht dessen haben sich die Erfinder die Aufgabe gestellt, einen Biomarker für Tierwohl zu entwickeln, der eine Unterscheidung zwischen kurz- und langfristiger Beeinträchtigung des Tierwohls erlaubt.

Diese Aufgabe wird von der vorliegenden Erfindung gelöst, die unten und in den Ansprüchen beschrieben ist.

Die Erfindung stellt ein Verfahren zur Bestimmung von Tierwohl zur Verfügung, bei dem eine Unterscheidung zwischen kurz- und langfristiger Beeinträchtigung des Tierwohls möglich ist , bei dem man die Expression von IGFBP-2 und IGFBP-3 in einer Probe eines Tieres analysiert, wobei das Verfahren zur Bestimmung der Transportzeit und/oder der Transportumstände eines Tieres eingesetzt wird, wobei eine niedrige Expression an IGFBP-2 und IGFBP-3 mit einer kurzfristigen Beeinträchtigung des Tierwohls, durch einen Transport des Tieres, assoziiert ist und wobei die Dauer des Stresses die Ausprägung der Veränderung der IGFBP-Konzentrationen verstärkt, wobei das Tier ein Schwein ist.

Bei Menschen ist bereits relativ gut belegt, dass Störungen des Growth Hormone (GH)-induzierten Wachstums an Auslenkungen der Konzentrationen von Insulin-Like Growth Factor I (IGF-I) sowie der IGF-Bindungsproteine (IGFBP)-2 und -3 erkannt werden können. Solche Störungen sind bei Menschen im Zusammenhang mit chronischer Krankheit oder akutem Stress auf zellulärer Ebene beschrieben (Messotten & van den Berghe, 2008). GH entfaltet seine anabolen Effekte einerseits direkt über den GH-Rezeptor, andererseits indirekt über eine Stimulation der Expression von IGF-I und IGFBP-3. Somit indizieren höhere Konzentrationen von IGF-I und IGFBP-3 das "normale" GH-induzierte Wachstum.

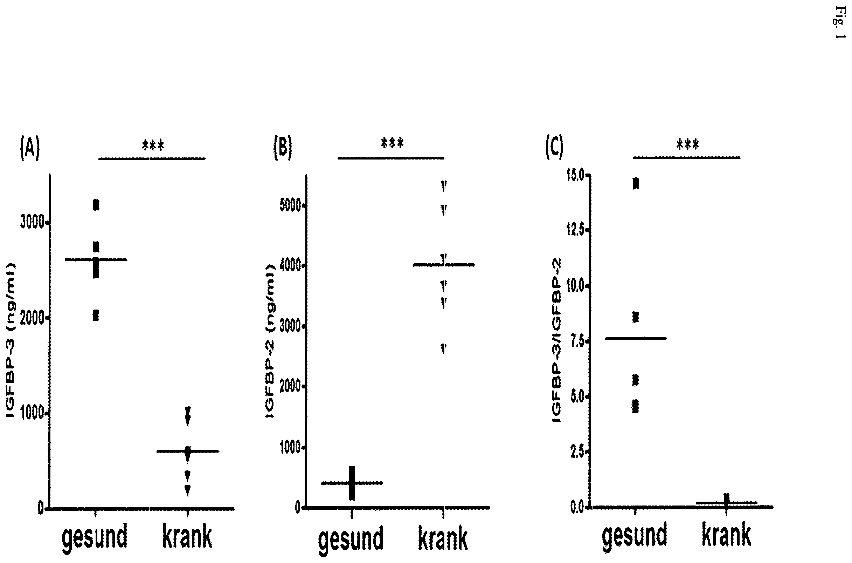

Menschliche Patienten, die akut erkrankt sind, haben signifikant niedrigere IGFBP-3-Konzentrationen als gesunde Probanden (Fig. 1A), wogegen die IGFBP-2 Konzentrationen der erkrankten Personen signifikant höher sind als die der Kontrollgruppe (Fig. 1B). Daraus ergibt sich bei der erkrankten Gruppe ein signifikant niedrigeres Verhältnis von IGFBP-3/IGFBP-2 (Fig. 1C). Das Verhältnis von IGFBP-3 zu IGFBP-2 ist daher beim Menschen ein sensitiver Parameter zur frühen Erkennung von Störungen der somatotropen Achse und damit verbundenen Krankheiten (Messotten und van den Berghe, 2008).

Wie Bielohuby et al. (2014) zeigen, gibt es jedoch deutliche Unterschiede in der Regulation des somatotrophen Systems, z.B. der Regulation von IGF-1, IGFBP-2 und IGFBP-3 zwischen Menschen und verschiedenen Tierarten, so dass eine direkte Übertragung der Ergebnisse nicht möglich ist.

Die Erfinder konnten zeigen, dass auch beim Schwein, ein erniedrigtes Verhältnis der Expression (bzw. Konzentration) von IGFBP-3 zu der Expression von IGFBP-2 mit einer Beeinträchtigung des Tierwohls assoziiert ist:.

Dies lässt sich auch in absoluten Werten für IGFBP-3 und IGFBP-2 ausdrücken: eine geringe Expression von IGFBP-3 und eine hohe Expression von IGFBP-2 ist mit einer langfristigen Beeinträchtigung des Tierwohls assoziiert. Allerdings zeigt sich dieses erniedrigte Verhältnis von IGFBP-3 zu IGFBP-2 erst bei einer langfristigen Beeinträchtigung des Tierwohls. Eine derartige Bestimmung einer langfristigen Beeinträchtigung des Tierwohls ist jedoch nicht durch die vorliegenden Ansprüche erfasst. Die folgende Beschreibung dient daher lediglich der Veranschaulichung.

Eine hohe Expression von IGFBP-3 und eine geringe Expression von IGFBP-2 ist hingegen mit Tierwohl assoziiert. Ein hohes Verhältnis der Expression von IGFBP-3 zur Expression von IGFBP-2 weist auf langfristiges Tierwohl hin. Die Bestimmung langfristigen Tierwohls ist ebenfalls nicht Teil der beanspruchten Erfindung.

Das Verhältnis oder die Höhe der Expression wird im Allgemeinen im Vergleich zu einer Vergleichsgruppe oder, bevorzugt, im Vergleich zu bekannten Vergleichswerten beurteilt. Diese Vergleichswerte sind durch Analyse von gesunden Kontrolltieren der gleichen Art, bevorzugt der gleichen Rasse zu ermitteln. Auch Alter, Masse, und Geschlecht sollten möglichst vergleichbar sein (z.B. Abweichungen von 30% oder weniger, 20% oder weniger, 10% oder weniger bei Alter und Masse). Dabei können für die Vergleichsgruppe bevorzugt standardisierte Haltungsbedingungen und sonstige Behandlung (z.B. keine Transporte, bevorzugt soweit möglich Vermeidung von Beeinträchtigung des Tierwohls) zugrunde gelegt, die z.B. bestimmten Richtlinien entsprechen können (z.B. Richtlinien eines Biosiegels). Es wird zur Bestimmung eines Vergleichswertes der Durchschnittswert bzw. eine gepoolte Probe einer Gruppe von Tieren (z.B. 5 oder mehr, 10 oder mehr, 20 oder mehr, 50 oder mehr) untersucht.

Von einem erniedrigten Verhältnis der Expression von IGFBP-3 zu der Expression von IGFBP-2 wird insbesondere ausgegangen, wenn dieses Verhältnis weniger als 60%, weniger als 50%, weniger als 40%, weniger als 30%, weniger als 20%, weniger als 10%, weniger als 5% des Vergleichswerts beträgt. Die Veränderungen sollten statistisch signifikant sein.

Eine langfristige Beeinträchtigung kann sich z.B. durch eine längerfristige Erkrankung, wie Lahmen, Mastitis, Mangelernährung, zu wenig Platz in der Haltung, schlechte Luft usw. ergeben. Üblicherweise treten solche Beeinträchtigungen über ein/e/n oder mehrere Woche/n, Monat/e oder Jahre hinweg auf, etwa 7 oder mehr Tage, 14 oder mehr Tage, 30 oder mehr Tage, 60 oder mehr Tage oder 360 oder mehr Tage.

Die Erfinder haben überraschenderweise festgestellt, dass eine niedrige Expression an IGFBP-2 und IGFBP-3 mit einer kurzfristigen Beeinträchtigung des Tierwohls assoziiert ist. Auch hier wird die Expression im Allgemeinen im Vergleich zu einer Vergleichsgruppe oder zu bekannten Vergleichswerten beurteilt.

Erfindungsgemäß ergibt sich die kurzfristige Beeinträchtigung durch Transport. Üblicherweise treten solche Beeinträchtigungen über ein oder mehrere Stunden oder bis zu wenigen Tagen auf, etwa 1 h bis zu 3 Tage, 2 h bis 2 Tage, 3 h - 1,5 Tag, 4-24 h, 6-24 h, 8-24 h, 12-24 h, 14-24 h, 16-24 h, 18-24 h, oder 20-24 h.

Das erfindungsgemäße Verfahren ermöglicht damit eine Differenzierung zwischen kurzfristiger und langfristiger Beeinträchtigung von Tierwohl, indem man die Expression von IGFBP-2 und IGFBP-3 bestimmt.

Die Erfindung stellt ein Verfahren zur Bestimmung von einer kurzfristigen Beeinträchtigung von Tierwohl durch einen Transport des Tieres zur Verfügung, bei dem man die Expression von IGFBP-2 und IGFBP-3 in einer Probe eines Tieres analysiert. Eine niedrige Expression an IGFBP-2 und IGFBP-3, bevorzugt im Vergleich zu einer Vergleichsgruppe oder einem Vergleichswert, ist mit einer kurzfristigen Beeinträchtigung des Tierwohls durch einen Transport des Tieres, assoziiert.

In einer Studie von McCusker et al. aus dem Jahr 1998 wurde gezeigt, dass Stress zu einer verringerten Expression von IGFBP-3 führt. (McCusker et al., 1998, Controlling Insulin-Like Growth Factor Activity and the Modulation of Insulin-Like Growth Factor Binding Protein and Receptor Binding. Journal of Dairy Science 81(6), 1790-1800). McCusker et al., 1989 offenbart, dass 24-stündiges Fasten zu einer Verminderung von IGFBP-2 und 3 in Plasma von Ferkeln führt (McCusker et al., 1989. The Insulin-Like Growth Factor-Binding Proteins of Porcine Serum: Endocrine and Nutritional Regulation. Endocrinology 125(1), 501-509). In Übereinstimmung mit dieser Feststellung beschreibt Laeger et al., 2014, dass eine kurzfristige Fastenperiode auch eine niedrigere Expression an IGFBP-2 und IGFBP-3 in Plasma von Kühen zur Folge hat (Laeger et al., 2014, Effects of parturition and feed restriction on concentrations and distribution oft he insulin-like growth factor-binding proteins in plasma abd cerebrospinal fluid of dairy cows. Journal of Dairy Science 97(5), 2876-2885). Zusammengefasst legt jedoch keines dieser Dokumente einen Zusammenhang zwischen einer niedrige Expression an IGFBP-2 und IGFBP-3 und einer kurzfristigen Beeinträchtigung des Tierwohls durch den Transport eines Tieres nahe.

Im Rahmen der Erfindung ist das Tier ein Schwein.

Die Probe kann ausgewählt sein aus der Gruppe umfassend Blut, Serum, Plasma, Seminal-Plasma, Zerebrospinalflüssigkeit, Follikelflüssigkeit, Urin, Sputum, Gewebe (z.B. Fleisch, etwa Muskelfleisch), Tropfsaft oder Milch. Während Untersuchungen beim Menschen im Allgemeinen auf Basis von Blutproben durchgeführt werden, konnten die Erfinder überraschenderweise zeigen, dass das erfindungsgemäße Verfahren auch auf Basis von Gewebeproben (z.B. Fleisch) oder Tropfsaft (Fleischsaft) funktioniert. Dies ermöglicht die Untersuchung insbesondere von Schweinen, nach der Schlachtung ohne zusätzliche Arbeitsschritte, wie Blutabnahme, am lebendigen Tier. Bevorzugt wird nach oder bei der Schlachtung abgenommenes Blut oder Fleisch untersucht.

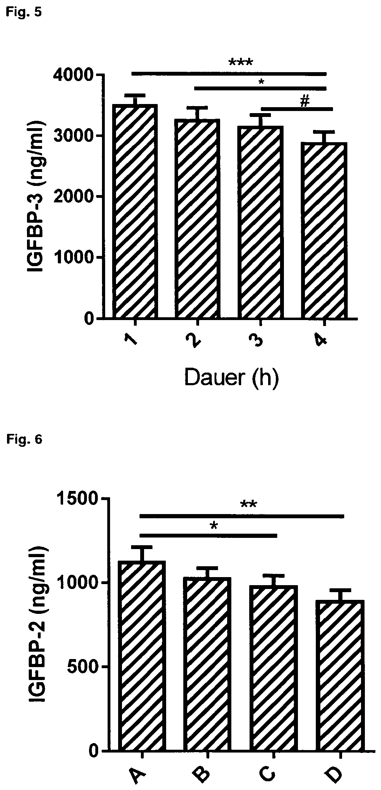

Insbesondere zur Untersuchung von kurzfristiger Beeinträchtigung des Tierwohls, kann die erfindungsgemäße Untersuchung gleich nach dem potentiell Stress auslösenden Ereignis, also gleich nach dem Transport (z.B. nach Ankunft im Schlachthof) durchgeführt werden. Die Dauer des Stresses kann die Ausprägung der Veränderung der IGFBP-Konzentrationen verstärken (siehe Fig. 5 und 6). Auch nach Schlachtung eines Tieres und Lagerung des Fleischs (möglichst gekühlt z.B. bei 4°C) oder der anderen Proben ist die Analyse möglich. Eine Probennahme nach Schlachtung ist bevorzugt, um zusätzliche Beeinträchtigung des Tierwohls zu vermeiden. Die IGFBPs können auch im Fleisch und Fleischsaft nachgewiesen werden, eine Messung zur Kontrolle von Tierwohl ist jederzeit möglich und kann selbst an der Ladentheke oder im Kühlregal erfolgen.

Es ist bei dem erfindungsgemäßen Verfahren selbstverständlich auch möglich, weitere Biomarker in der Probe zu analysieren. Z.B. kann man ferner die Expression von IGF-I in der Probe analysieren. Eine höhere Expression von IGF-I ist mit Tierwohl assoziiert. Auch hier findet die Beurteilung üblicherweise mit einem geeigneten Vergleichswert statt.

Mit dem erfindungsgemäßen Verfahrens kann ferner mit geeigneten Maßnahmen - zumindest stichprobenartig - kontrolliert werden, ob eine exogene Gabe anaboler Hormone (z.B. GH oder IGF-1) stattfand. Eine solche Gabe exogener Hormone könnte die Ergebnisse des erfindungsgemäßen Verfahrens verfälschen. Allerdings führt die Gabe von GH/IGF-I zu weit stärkeren Auslenkungen des somatotropen Systems als unter haltungsbedingten Effekten zu erwarten wäre. Daher ist eine unbemerkte Verfälschung wenig wahrscheinlich. Der Einsatz von GH/IGF in der Tierhaltung kann durch nicht angekündigte vor Ort Kontrollen und/oder Blutproben ausgeschlossen werden. Die Verwendung der IGFBP-Signatur soll auch als Mittel eingesetzt werden um spezifische Haltungsbedingungen zu bewerten und dabei das Tierwohl zu verbessern. In der EU ist die Gabe exogener anaboler Hormone zur Leistungsförderung bei Nutztieren wie Schweinen und Rindern aktuell verboten, in den USA z.B. jedoch erlaubt.

Die Expression der erfindungsgemäßen Biomarker IGFBP-2 und IGFBP-3 kann z.B. mit dem von Hossenlopp et al., 1987 beschriebenen Verfahren vergleichend untersucht werde, allerdings liefert dieses Verfahren keine quantitativen Daten. ELISA oder RIA sind jedoch unzuverlässig aufgrund mangelnder Spezifität für intakte IGFBPs. Bevorzugt analysiert man die Expression der erfindungsgemäßen Biomarker auf Protein-Ebene daher mit einem quantitativen Western Liganden Blot. Die Erfinder haben gezeigt, dass sich mit diesem Verfahren deutlich genauere Ergebnisse erhalten lassen als bei im Stand der Technik im allgemeinen verwendeten ELISAs, da die dort eingesetzten Antikörper auch an nicht-funktionelle Fragmente der Bindungsproteine binden.

Zur Analyse der IGF-Bindungsproteine werden die Proteine beim quantitativen Western Liganden Blot mittels SDS-PAGE aufgetrennt und anschließend auf eine Membran, z.B. Nitrocellulose oder, bevorzugt PVDF, transferiert. Die Membran wird geblockt und anschließend mit markiertem IGF (z.B. mit Biotin markiert), insbesondere IGF-2 inkubiert. Nach mehreren Waschschritten folgt eine Inkubation mit einem Nachweisreagenz, im Falle von Biotin etwa ein Streptavidin-Konjugat. Auch ein Avidin-Konjugat kann verwendet werden. Bevorzugt wird ein Streptavidin-Peroxidase-Konjugat eingesetzt. Nach erneutem Waschen und Zugabe eines Substrats werden die IGF-Bindungsproteine z.B. durch Chemilumineszenz detektiert und analysiert, z.B. mit einer KODAK IMAGE Station aufgenommen. Die Quantifizierung erfolgt durch Kalibrierkurven, z.B. mit rekombinanten humanen IGFBPs oder, optional, rekombinanten IGFBPs der untersuchten Spezies, z.B. Schwein. Die Kurvenanpassungen der einzelnen Standards erfolgt bevorzugt durch eine vierparametrische nichtlineare Regression.

Um die unterschiedliche Transfereffizienz und Auswirkung von Artefakten der Aufarbeitung (waschen, blockieren, Inkubieren) zu eliminieren, wird bevorzugt eine interne Standardisierung vorgenommen, d.h., die Probe wird mit einem internen Standard versetzt. Hierzu kann entweder ein IGFBP mit einem von den zu untersuchenden IGFBPs unterscheidbaren Molekulargewicht (bevorzugt 50-100 kDa) aus der gleichen oder einer anderen Spezies verwendet werden (das können künstliche IGFBPs sein, die über einen Klonierungsvektor vergrößert oder verkleinert wurden oder heterolgoe IGFBPs aus anderen Spezies mit unterscheidbaren Molekulargewichten, bevorzugt IGFBP-2), oder ein beliebiges biotinyliertes Protein jedweder anderen Klasse, welches den Nachweis nicht stört und ein geeignetes Molekulargewicht aufweist (Nicht-IGFBP, vorzugsweise: 50-100 kDa). Es kann vorteilhafterweise die Fähigkeit des unterscheidbaren IGFBP ausgenutzt werden, an IGF, z.B. IGF-2-Biotin, zu binden, so dass der Nachweis auf den gleichen Voraussetzungen beruht wie der Nachweis von IGFBP-2 und IGFBP-3.Dadurch ergibt sich eine besondere Genauigkeit des Assays. Der interne Standard wird dann über das Assay-spezifische Detektionssystem (z.B. Streptavidin-gekoppelte Peroxidase = "Konjugat") nachgewiesen.

Alternativ kann bei dem erfindungsgemäßen Verfahren die Expression auf RNA-Ebene analysiert werden, wobei z.B. aus Fleisch aufgereinigt werden kann.

In einer Ausführungsform kann man in dem erfindungsgemäßen Verfahren das Tierwohl einer Population bestimmen, mit deren Tieren unter untereinander gleichen oder vergleichbaren Bedingungen umgegangen wurde. Dabei kann man optional ferner das Tierwohl einer weiteren Population bestimmen, mit deren Tieren unter anderen untereinander gleichen oder vergleichbaren Bedingungen umgegangen wurden, wobei man das Tierwohl der Populationen vergleicht. "Ein" bedeutet im Rahmen der Erfindung "ein oder mehrere", sofern dies nicht ausgeschlossen ist. Es können also z.B. auch mehr als zwei Populationen miteinander verglichen werden. Am aussagekräftigsten ist dieser Vergleich, wenn Tiere der gleichen Rasse und möglichst ähnlichen Alters oder Geschlechts (oder möglichst ähnlicher Verteilung dieser Parameter) verglichen werden. Unter "Umgang" werden hierin Transporte verstanden.

Die Erfindung stellt auch ein Verfahren zur Optimierung des Umgangs mit einem Tier bereit, d.h., der Transportbedingungen eines Tieres, bei dem man das erfindungsgemäße Verfahren einsetzt. Dabei können potentielle Beeinträchtigungen des Tierwohls erkannt werden, und man kann versuchen, den Umgang, d.h. die Transportbedingungen so anzupassen, dass das Tierwohl verbessert wird.

Die Erfindung stellt somit auch ein Verfahren zur Bestimmung der Transportzeit und/oder der Transportumstände eines Tieres bereit, bei dem man das erfindungsgemäße Verfahren einsetzt.

In einem anderen Aspekt stellt die Erfindung die Verwendung eines Kits zur Bestimmung von Tierwohl mit dem erfindungsgemäßen Verfahren zur Verfügung, welches Standards zur Quantifizierung von IGFBP-3 und IGFBP-2 sowie ein markiertes IGF umfasst, welches in der Lage ist, an IGFBP-2 und IGFBP-3 zu binden, z.B. IGF-2 (welches ggü. IGF-1 eine erhöhte Sensitivität ermöglicht). Bevorzugt umfasst das Kit IGF-2-Biotin. Es kann auch Mittel zur Durchführung eines quantitativen Western Blots, umfassen. Mittel zur Durchführung eines quantitativen Western Blots könnten z.B. sein: eine PVDF-Membran, Waschpuffer, Blockierungspuffer, Substratlösung (bzw. Konzentrate für Puffer und Lösungen). Auch ein Konjugat zum Nachweis des markierten IGF-Biotin, z.B. Streptavidin-Peroxidase, kann im Kit enthalten sein. Das Kit kann ferner ein zur internen Standardisierung geeignetes Protein umfassen, z.B. ein IGFBP mit einem Molekulargewicht, welches von den zu untersuchenden IGFBP-2 und IGFBP-3 unterscheidbar ist. Dieses IGFBP kann einer anderen Spezies angehören als die als Standard eingesetzten IGFBP-2 und IGFBP-3. Wenn es sich bei dem zur internen Standardisierung geeignetes Protein nicht um ein IGFBP handelt, kann das Protein mit Biotin markiert sein und hat bevorzugt ein Molekulargeweicht von 50-100 kDa.
**Fig. 1****: Konzentrationen von IGFBP-3 (A), IGFBP-2 (B) und deren Verhältnis (C) in gesunden und erkrankten Menschen. Modifiziert nach Messotten and van den Berghe, 2008.**
**Fig. 2****: Exemplarische IGFBP-Profile in biologischen Flüssigkeiten aus Schweinen: (A) Serum, (B) Seminal-Plasma (C) Zerebrospinalflüssigkeit (D) Follikelflüssigkeit**
**Fig. 3****: IGFBP-3 Serum Konzentrationen von weiblichen Schweinen der Rassen Mangalitza und Deutsche Landrasse (A)**
**Fig. 4** **Versuchsdesign einer Pilotstudie zu Beeinträchtigung von Tierwohl bei Transporten.**
**Fig. 5** **Messung von IGFBP-3 bei der Pilotstudie (A)** Die Konzentration an IGFBP-3 (ng/ml) im Vergleich zu Probe A (vor dem Transport) sinkt bereits 2 h nach dem Transport (B) sowie weiteren 2 h nach dem Aufenthalt in der Wartebucht (C) bzw. nach Schlachtung ( D) signifikant (*: p<0.05; **:p<0.01).
**Fig. 6** **Messung von IGFBP-2 bei der Pilotstudie (A)** Die Konzentration an IGFBP-2 (ng/ml) im Vergleich zu Probe A sinkt in Abhängigkeit der Zeit nach dem Transport bei den Proben B, C und D signifikant (*p<0.05; **p<0.01).
**Fig. 7** **Kommerzieller Schweinetransport in Canada**
**Fig. 8 Auswirkungen von Transport über 6 h, 12 h oder 18 h auf die Konzentration von IGFBP-2 (A) und IGFBP-3 (B).** Es wurden 80 Schweine pro Gruppe untersucht (*: p<0.05; #: p<0.05; ***:p<0.001).

### Beispiele

### Quantitativer Western Liganden Blot

Zur Analyse der IGF-Bindungsproteine wurden die Serum-Proteine mittels SDS-PAGE aufgetrennt und anschließend auf eine PVDF-Membran transferiert. Die Membran wurde für 20 min mit Quenching-Lösung inkubiert, dann für 1 h blockiert und anschließend für 2 h mit Biotin-markiertem IGF-2 (ibt-Systems) in Blockierungspuffer (5 µg/ml) inkubiert. Nach 5x 5 min Waschen folgte eine Inkubation für 1 hmit einem Streptavidin-Peroxidase-Konjugat. Nach erneutem Waschen (5x5 min) und Substratzugabe (10 ml Lumina Forte (Milipore), 4 min lichtgeschützt) wurden die IGF-Bindungsproteine durch Chemilumineszenz detektiert und mit der KODAK IMAGE Station aufgenommen. Die Quantifizierung erfolgte durch Kalibrierkurven mit rekombinanten humanen IGFBPs. Die Kurvenanpassungen der einzelnen Standards erfolgten durch eine vierparametrische nichtlineare Regression. Alle Wasch- und Inkubationsschritte erfolgen bei Schütteln, z.B. auf einem Kippschüttler bei RT (ca. 20-25°C).

### Untersuchung von porcinen Proben

Es wurden Proben von weiblichen Tieren der Rassen Mangalitza (n = 10, Alter 1-2 Jahre) und Deutsche Landrasse (n = 12, Alter < 1 Jahr) analysiert. Die Proben der Mangalitza Rasse stammen aus dem Research Institute for Animal Breeding, Nutrition and Meat Science, Herceghalom in Ungarn. Die Tiere der Deutschen Landrasse stammen aus der Experimentalanlage Schwein, FBN Dummerstorf. Der Vergleich der Mittelwerte erfolgte mit Hilfe des t-Tests, wobei das Signifikanzniveau α auf 0,05 festgelegt wurde.

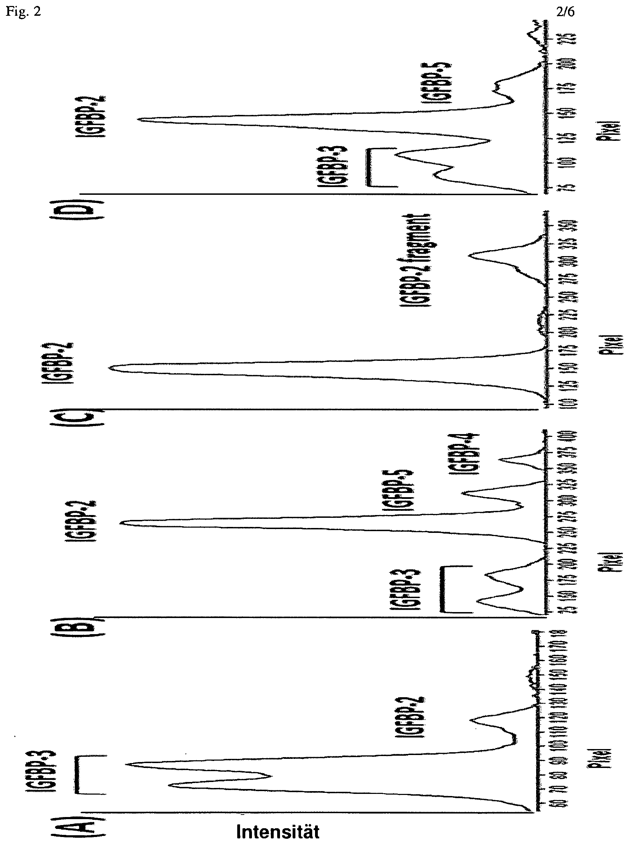

Über den modifizierten quantitativen Western-Liganden-Blot können alle IGFBPs visualisiert werden, die in einer gegebenen Probe oder Matrix vorhanden sind (Fig. 2A-D). Durch den quantitativen Charakter der Daten können auch größere Untersuchungsgruppen verglichen werden. Die aktuelle Methode ist ein wertvolles Mittel um in verschiedenen Matrices von Nutztieren den Status der somatotropen Achse zu beurteilen.

Auch beim Schwein ist IGFBP-3 das dominante IGF-Bindungsprotein, gefolgt von IGFBP-2. Damit scheinen sich zumindest auf Ebene der relativen Expression die Verhältnisse der IGF-Bindungsproteine zwischen Mensch und Schwein zu ähneln. Somit ist eine Voraussetzung erfüllt, um mögliche Störungen der somatotropen Achse auch beim Schwein zu erkennen.

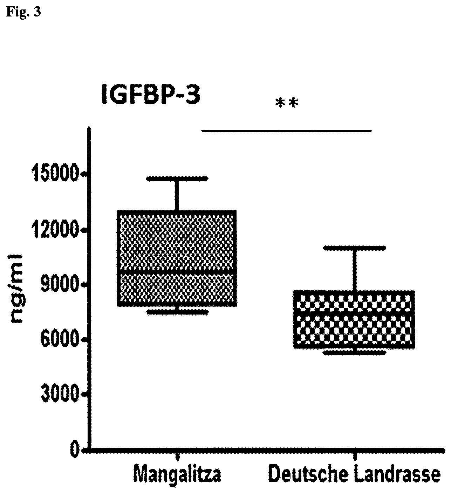

Die untersuchten Mangalitza Schweine weisen zudem signifikant höhere IGFBP-3 Konzentrationen im Serum auf als Tiere der Deutschen Landrasse (Fig. 3A). Das Verhältnis von IGFBP-3/IGFBP-2 ist bei den Mangalitza Schweinen tendenziell höher als bei den Tieren der Deutschen Landrasse (p < 0,1), was auf ein höheres Tierwohl hindeuten könnte. Die Mangalitza Schweine zeigen einen hohen Fettansatz, langsames Wachstum, Spätreife und sind extensiv gehalten. Die Schweine der Deutschen Landrasse zeigen hohen Muskelansatz, schnelles Wachstum, Frühreife und sind intensiv gehalten. Es ist jedoch auch möglich, dass Rasse- oder Alterseffekte die unterschiedlichen Konzentrationen an IGFBP-3 verursacht haben (Fig. 3B). Eine größere Aussagekraft lässt sich durch Vergleich von Populationen gleicher Rasse, und möglichst auch gleichen Alters erzielen.

### Pilotstudie zur Auswirkung von Transporten auf das Tierwohl

40 Versuchstiere (Schweine, Dänische Landrasse x Pietrain) wurde eine Probe A entnommen. Dann wurden sie in der Schweinemastanlage Karrenzin verladen, zwei Stunden zum VION Schlachtbetrieb in Perleberg transportiert und abgeladen. Nach 2 Stunden Wartezeit, am Tag des Transports, erfolgte die Schlachtung. Probe B wurde direkt nach dem Transport entnommen, Probe C nach der Wartezeit. Proben D und E (Gewebeprobe) wurden direkt nach der Schlachtung entnommen, Probe F (Gewebe) am nächsten Tag ca. 24 h nach Schlachtung, nach Lagerung bei 4°C. Proben A-D waren Blutproben. Das Gewicht bei Versuchsbeginn war ca. 120 kg. Von 40 Kontrolltieren wurden nach Schlachtung Blutprobe asserviert und Gewebe entnommen.

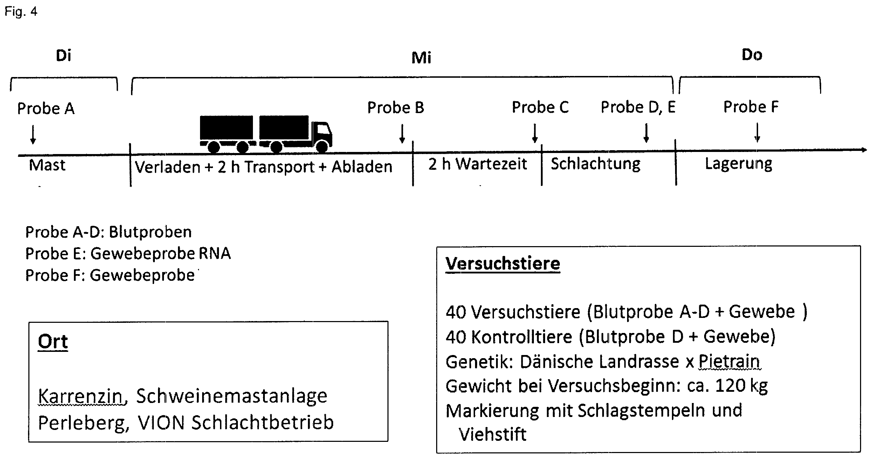

Das Schema ist in Fig. 4 dargestellt, die Ergebnisse in Fig. 5 und 6. Diese Ergebnisse zeigen, dass durch den Transport die Konzentration sowohl von IGFBP-2 als auch von IGFBP-3 signifikant abnehmen. Dies ist nicht nur direkt nach dem Transport nachweisbar, sondern auch noch bei Probenentnahme mehrere Stunden nach dem Transport und nach der Schlachtung. Auch nach mehr als 12 h nach der Schlachtung war der Nachweis der durch den Transport erfolgten Beeinträchtigung des Tierwohls auch Basis der Biomarker IGFBP-2 und IGFBP-3 noch nachweisbar.

### Demonstratorprojekt zu Auswirkungen von kommerziellem Schweinetransport auf das Wohlbefinden

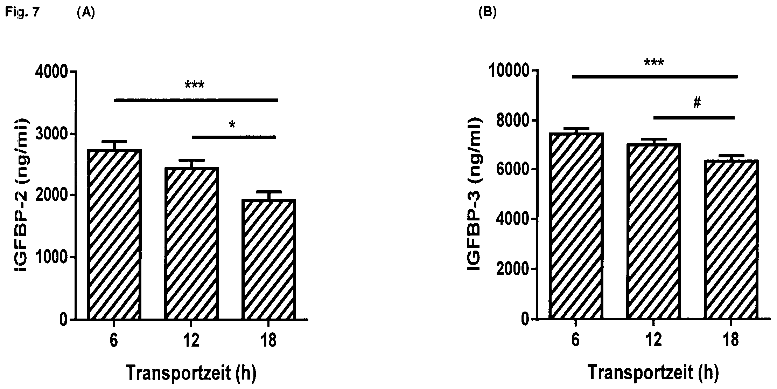

Das Projekt wurde in Kooperation mit dem Dairy and Swine Reaserach and Development Centre, Canada, durchgeführt. 240 Schweine (Landrasse x Edelschwein) mit durchschnittlichem Schlachtgewicht von 120,8 +- 0,4 kg wurden für 6h, 12 h oder 18 h transportiert (Fig. 7). Die Ergebnisse sind in Fig. 8 dargestellt.

Diese Ergebnisse zeigen eine deutliche Assoziation zwischen der Transportzeit, die zu einer Beeinträchtigung des Tierwohls führt, und einer von der Zeit der Beeinträchtigung abhängigen Abnahme der Expression von IGFBP-2 und IGFBP-3

### Literatur

Bielohuby M. (2014). Validation of serum IGF-I as a biomarker to monitor the bioactivity of exogenous growth hormone agonsits and antagonists in rabbits. Disease Models & mechanisms 7:1263-1273.

Broom, D.M. (1986): Indicators of poor welfare. Br. Vet. J. 142: 524-526.

Broom, D.M. (1991): Animal welfare: Concepts and Measurement. J. Anim. Sci 69: 4167-4175

Goumon S, Brown JA, Faucitano L, Bergeron R, Widowski TM, Crowe T, Connor ML, Gonyou HW. (2013): Effects of transport duration on maintenance behavior, heart rate and gastrointestinal tract temperature of market-weight pigs in 2 seasons. J Anim Sci. 91: 4925-4935.

Hoeflich A, Wirthgen E, David R, Classen CF, Spitschak M, Brenmoehl J. (2014): Control of IGFBP-2 Expression by Steroids and Peptide Hormones in Vertebrates. Front. Endocrinol. (Lausanne). 5:43. doi: 10.3389/fendo.2014.00043.

Mesotten D. and Van den Berghe G. (2008): Changes within the GH/IGF-I/IGFBP axis in critical illness. In: Contemporary Endocrinology: Acute Cause to Consequence Edited by: G. Van den Berghe, DOI: 10.1007/978-1-60327-177-6 10, C. Humana Press, New York, NY.

Mysegades, W. (2014). Untersuchungen zur Eignung des IGF-I Systems zur Prognose der Gesundheit und Fertilität bei pluriparen Deutschen Holstein Milchkühen. Dissertation an der Tierärztlichen Hochschule Hannover.

Ruffer, U.,(2003). Einflussfaktoren auf die IGF-1-Konzentrationen in Viertelanfangsmelkproben von Kühen mit unterschiedlicher Eutergesundheit - eine Feldstudie. Dissertation an der Justus-Liebig-Universität Gießen, Selbstverlag des Instituts für Tierzucht und Tierhaltung der Christian-Albrechts-Universität zu Kiel.

Walker SL et al. (2008) Chronic stress, hormone profiles and estrus intensity in dairy cattle. Hormones and Behaviour 53: 493-501.

## Patentansprüche

1. Verfahren zur Bestimmung von Tierwohl, bei dem eine Unterscheidung zwischen kurzfristiger und langfristiger Beeinträchtigung des Tierwohls möglich ist, bei dem man die Expression von IGFBP-2 und IGFBP-3 in einer Probe eines Tieres analysiert, wobei das Verfahren zur Bestimmung der Transportzeit und/oder der Transportumstände eines Tieres eingesetzt wird, wobei eine niedrige Expression im Vergleich zu einer Vergleichsgruppe oder, bevorzugt, im Vergleich zu bekannten Vergleichswerten an IGFBP-2 und IGFBP-3 mit einer kurzfristigen Beeinträchtigung des Tierwohls, durch einen Transport des Tieres, assoziiert ist und wobei die Dauer des Stresses die Ausprägung der Veränderung der IGFBP-Konzentrationen verstärkt, wobei das Tier ein Schwein ist,
wobei eine kurzfristige Beeinträchtigung durch Transport über etwa 1 h bis zu 3 Tage auftritt, während eine langfristige Beeinträchtigung über etwa 7 oder mehr Tage auftritt.

2. Verfahren nach Anspruch 1, wobei die Probe ausgewählt ist aus der Gruppe umfassend Blut, Serum, Plasma, Seminal-Plasma, Zerebrospinalflüssigkeit, Follikelflüssigkeit, Urin, Sputum, Gewebe, Tropfsaft oder Milch.

3. Verfahren nach einem der vorangehenden Ansprüche, wobei die Untersuchung nach der Schlachtung erfolgt und die Probe Fleisch oder Fleischsaft ist.

4. Verfahren nach einem der vorangehenden Ansprüche, wobei man ferner die Expression von IGF-I und/oder weiterer IGFBPs in der Probe analysiert.

5. Verfahren nach einem der vorangehenden Ansprüche, wobei man die Expression auf Protein-Ebene mit einem quantitativen Western Liganden Blot analysiert oder auf RNA-Ebene analysiert.

6. Verfahren nach einem der vorangehenden Ansprüche, wobei man das Tierwohl einer Population bestimmt, mit deren Tieren unter untereinander gleichen oder vergleichbaren Bedingungen umgegangen wurde,
wobei man optional ferner das Tierwohl einer weiteren Population bestimmt, mit deren Tieren unter anderen untereinander gleichen oder vergleichbaren Bedingungen umgegangen wurden, wobei man das Tierwohl der Populationen vergleicht.

7. Verwendung eines Kits, das Standards zur Quantifizierung von IGFBP-3 und IGFBP-2, und markiertes IGF umfasst, in einem Verfahren nach einem der vorangehenden Ansprüche.

8. Verfahren nach einem der Ansprüche 1 - 6 zur Optimierung des Umgangs mit einem Tier.

9. Verfahren nach einem der Ansprüche 1 - 6 zur Etablierung eines Tierwohlstandards und/oder von Qualitätsstufen bei tierischen Produkten.

## Claims

1. A method of determining animal welfare, wherein a distinction between short-term and long-term impairment of the animal welfare is possible, wherein the expression of IGFBP-2 and IGFBP-3 in a sample from an animal is analyzed, wherein the method is used to determine transport duration and/or transport conditions of an animal, wherein a low expression in comparison to a reference group or, preferably, in comparison to known reference values of IGFBP-2 and IGFBP-3, is associated with a short-term impairment of animal welfare, due to transport of the animal, and wherein the duration of stress increases the extent of the change in the IGFBP concentrations, wherein the animal is a pig, wherein a short-term impairment occurs due to transport over approximately 1 hour to 3 days, while a long-term impairment occurs over approximately 7 or more days.

2. The method of claim 1, wherein the sample is selected from the group comprising blood, serum, plasma, seminal plasma, cerebrospinal fluid, follicular fluid, urine, sputum, tissue, drip fluid, or milk.

3. The method of any of the preceding claims, wherein the analysis is performed after slaughter and the sample is meat or meat juice.

4. The method of any of the preceding claims, wherein the expression of IGF-1 and/or additional IGFBPs in the sample is further analysed.

5. The method of any of the preceding claims, wherein the expression is analysed on a protein level with a quantitative Western Ligand Blot, or analysed on a RNA level.

6. The method of any of the preceding claims, wherein the animal welfare of a population is determined, whose animals have been treated with internally the same or comparable conditions, wherein optionally the animal welfare of an additional population is further determined, whose animals have been treated with other, internally the same or comparable conditions, wherein the animal welfare of the populations is compared.

7. A use of a kit comprising standards for quantification of IGFBP-3 and IGFBP-2 and marked IGF, in a method of any of the preceding claims.

8. The method of any of the claims 1-6 for optimization of the handling of an animal.

9. The method of any of the claims 1-6 for establishing an animal welfare standard and/or quality indicators of animal products.

## Revendications

1. Procédé de détermination du bien-être d'un animal, dans lequel une distinction peut être faite entre une atteinte à court terme et une atteinte à long terme au bien-être de l'animal, dans lequel l'expression d'IGFBP-2 et d'IGFBP-3 dans un échantillon provenant d'un animal est analysée, dans lequel le procédé est utilisé pour la détermination de la durée de transport et/ou des conditions de transport d'un animal, dans lequel une expression faible par rapport à un groupe de référence ou, de préférence, par rapport à des valeurs de référence connues d'IGFBP-2 et d'IGFBP-3, est associée à une atteinte à court terme au bien-être de l'animal pendant un transport de celui-ci et dans lequel la durée du stress amplifie l'effet de la modification des concentrations en IGFBP, dans lequel l'animal est un porc, dans lequel une atteinte à court terme pendant le transport survient au cours d'entre environ 1 heure et 3 jours, tandis qu'une atteinte à long terme survient au cours d'environ 7 jours ou plus.

2. Procédé selon la revendication 1, dans lequel l'échantillon est choisi dans le groupe comprenant le sang, le sérum, le plasma, le plasma séminal, le liquide cérébrospinal, le liquide folliculaire, l'urine, l'expectoration, les tissus, l'exsudat ou le lait.

3. Procédé selon l'une des revendications précédentes, dans lequel l'analyse se produit après l'abattage et l'échantillon est de la chair ou un liquide extrait de la chair.

4. Procédé selon l'une des revendications précédentes, dans lequel l'expression d'IGF-I et/ou d'IGFBP supplémentaires est en outre analysée dans l'échantillon.

5. Procédé selon l'une des revendications précédentes, dans lequel le taux d'expression des protéines est analysé par Western blot quantitatif ou le taux d'expression de l'ARN est analysé.

6. Procédé selon l'une des revendications précédentes, dans lequel le bien-être d'une population d'animaux, dont les animaux sont traités dans des conditions identiques ou comparables les unes aux autres, est déterminé,
facultativement, dans lequel le bien-être d'une population d'animaux supplémentaire, dont les animaux sont traités dans d'autres conditions identiques ou comparables les unes aux autres, est en outre déterminé, dans lequel le bien-être des populations d'animaux est comparé.

7. Utilisation d'un kit qui comprend des étalons permettant la quantification d'IGFBP-3 et d'IGFBP-2 et de l'IGF marqué dans un procédé selon l'une des revendications précédentes.

8. Procédé selon l'une des revendications 1 à 6 permettant l'optimisation du traitement d'un animal.

9. Procédé selon l'une des revendications 1 à 6 permettant la mise en place d'une norme de bien-être animal et/ou de niveaux de qualité de produits d'origine animale.
